(19) Europäisches Patentamt European Patent Office Office européen des brevets

(11) **EP 1 927 846 A1**

(12) **DEMANDE DE BREVET EUROPEEN**

(43) Date de publication:
**04.06.2008 Bulletin 2008/23**

(51) Int Cl.:
***G01N 15/08*** *(2006.01)*     *G01N 33/24* *(2006.01)*

(21) Numéro de dépôt: **07291405.4**

(22) Date de dépôt: **23.11.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **01.12.2006 FR 0610581**

(71) Demandeur: **IFP**
**92852 Rueil-Malmaison Cédex (FR)**

(72) Inventeurs:
• **Egermann, Patrick**
  **92500 Rueil Malmaison (FR)**
• **Le Ravalec, Mickaele**
  **92500 Rueil Malmaison (FR)**
• **Soltani, Amir**
  **92400 Courbevoie (FR)**

(54) **Méthode pour caractériser la distribution de la perméabilité absolue d'un échantillon hétérogène**

(57)     - L'invention concerne une méthode de détermination de la distribution tridimensionnelle de la perméabilité absolue d'un échantillon hétérogène, dans laquelle on effectue les étapes suivantes:

- on détermine une carte de porosité tridimensionnelle 3D de l'échantillon,
- on réalise une expérience de déplacement miscible visqueux au cours de laquelle on détermine l'évolution de la pression différentielle de part et d'autre de l'échantillon,
- à partir de cette évolution, on détermine un profil 1D de perméabilité absolue le long de l'échantillon. Ensuite, on construit une première carte 3D de perméabilité à partir de la carte 3D de porosité,
- on estime une pression différentielle simulée en simulant numériquement un test miscible visqueux à partir de la première carte de perméabilité et du profil 1D de perméabilité,
- on détermine la distribution tridimensionnelle de la perméabilité absolue de l'échantillon en modifiant au moins une fois la première carte de perméabilité de façon à minimiser l'écart entre la pression différentielle simulée et la pression différentielle mesurée au cours du temps.

- Application à l'exploration pétrolière et au stockage de $CO_2$.

**Fig. 8**

**Description**

La présente invention concerne le domaine des mesures pétrophysiques sur des échantillons de roches.
Plus particulièrement, l'invention concerne le domaine des procédés permettant de caractériser la distribution tridimensionnelle de la perméabilité absolue d'un échantillon hétérogène issu d'une roche.

**État de la technique**

On connaît différentes approches pour caractériser une carte tridimensionnelle (3D) de perméabilité d'un échantillon d'une roche.

*Identification d'une carte 3D basée sur une identification "manuelle"*

Cette approche consiste à utiliser un test de traceurs, c'est-à-dire un test miscible mais sans contraste de viscosité, pour obtenir une carte de perméabilité. Cette technique est décrite dans les documents suivants :

- Dabbouk, C., Ali, L., Williams, G., Beattie, G. :"Waterflood in vuggy layer in a Middle East reservoir - Displacement physics understood", SPE 78530, Abu Dhabi International Petroleum Exhibition and Conference, 13-16 October 2002.
- Olivier, P., Cantegrel, L., Laveissière, J., Guillonneau, N. : "Multiphase flow behaviour in vugular carbonates using X-Ray", SCA 2004-13, Society of Core Analysts Symposium, Abu Dhabi, 5-9 October 2004.

Une ou plusieurs lois K(Φ) sont utilisées pour déduire d'une carte de porosité, une carte de perméabilité servant à la simulation de l'expérience de traceurs. Ces lois K(Φ) sont alors ajustées de façon manuelle jusqu'à obtenir un bon accord avec l'expérience. L'avantage de cette approche est sa simplicité. En revanche, un inconvénient majeur est qu'il existe un très grand nombre de solutions à ce problème. Or, un calage manuel ne donne accès qu'à une seule solution.

*Identification d'une carte 3D basée sur une inversion "mathématique"*

Cette technique s'inscrit toujours dans le cadre des tests de traceurs. Selon cette méthode, il est possible de suivre, par exemple par tomographie, le déplacement d'un front à différents temps. A partir de cette information, la carte de perméabilité est solution d'un problème non linéaire avec des conditions aux limites connues. Cette méthode est décrite dans :

- Zhan, L., Yortsos, Y.C. :"A direct method for the identification of the permeability field of an anisotropic porous medium", SPE 62976, Annual Technical Conference and Exhibition, Dallas, 1-4 October 2000.

Dans la mesure où les conditions aux limites (profil de pression ou perméabilité sur les frontières) et la carte de porosité sont connues, cette technique conduit à une solution unique, qui n'est valide que lorsque les contrastes de perméabilité sont faibles. La mise en oeuvre de cette technique reste donc limitée en pratique, car les conditions aux limites ne sont pas toujours connues. Cette méthode ne s'applique pas aux milieux très hétérogènes.

*Profil 1D basé sur un déplacement visqueux*

La mise en oeuvre d'un déplacement visqueux pour obtenir des informations sur le profil 1D de perméabilité a été proposée par Fincham et Gouth :

- Fincham, A., Gouth, F. :"Improvements of coreflood design and interpretation using a new software", SCA 2000, Society of Core Analysts Symposium, Abu Dhabi, 9-12 October 2000.

Ces auteurs proposent d'interpréter le signal de pression différentielle d'une injection d'huile visqueuse à fort débit dans un massif initialement saturé en saumure pour déterminer le profil 1D de perméabilité à l'huile à $S_{wi}$. Grâce à cette technique les auteurs réussissent à mieux caractériser l'hétérogénéité "locale" de leur échantillon et ainsi à affiner l'interprétation des expériences visant à la détermination des courbes de perméabilités relatives. Cependant, cette approche ne permet pas de remonter aux valeurs des perméabilités absolues que ce soit en 1D ou en 3D.
L'objet de la présente invention concerne une méthode permettant de caractériser de façon rapide, précise et non destructive, la distribution tridimensionnelle de la perméabilité absolue d'un échantillon hétérogène quelconque.
L'invention concerne une méthode de détermination d'une distribution tridimensionnelle de la perméabilité absolue d'un échantillon hétérogène, à partir d'une carte de porosité tridimensionnelle dudit échantillon, dans laquelle on effectue les étapes suivantes :

- on réalise au moins une expérience de déplacement miscible visqueux au cours de laquelle on détermine une évolution de la pression différentielle $DP(t)_{mes}$ de part et d'autre dudit échantillon ;

- on détermine un profil 1D de perméabilité absolue le long dudit échantillon en utilisant ladite évolution de la pression différentielle ;

- on construit une première carte 3D de perméabilité $(K(\Phi))$ à partir de ladite carte 3D de porosité ;

- on estime une pression différentielle simulée $DP(t)_{sim}$, en simulant numériquement un test miscible visqueux à partir de ladite première carte de perméabilité et dudit profil 1D de perméabilité ;

- on détermine ladite distribution tridimensionnelle de la perméabilité absolue dudit échantillon en modifiant au moins une fois ladite première carte de perméabilité de façon à minimiser l'écart entre ladite pression différentielle simulée $DP(t)_{sim}$ et ladite pression différentielle mesurée au cours du temps $DP(t)_{mes}$.

On peut déterminer ladite carte de porosité à partir de mesures statiques non destructives.

On peut déterminer ladite carte de porosité à partir de mesures par rayons X en utilisant des fluides de densités différentes saturant ledit échantillon.

On peut déterminer ladite carte de porosité à partir de mesures par résonance magnétique nucléaire.

On peut déterminer ladite première carte de perméabilité $(K(\Phi))$ à partir de co-simulations.

On peut déterminer ladite première carte de perméabilité $(K(\Phi))$ à partir d'au moins une relation déterministe entre la porosité et la perméabilité.

On peut modifier ladite première carte de perméabilité en appliquant une méthode de déformation graduelle.

On peut déterminer en outre un profil de concentration ou des cartes 3D de concentration à des temps différents, que l'on compare à des réponses simulées équivalentes $C_{sim}$.

D'autres caractéristiques et avantages de la méthode selon l'invention, apparaîtront à la lecture de la description ci-après d'exemples, non limitatifs, de réalisations, en se référant aux figures annexées et décrites ci-après.

**Présentation succincte des figures:**

La figure 1 illustre le dispositif expérimental permettant de mettre en oeuvre la méthode selon l'invention.

La figure 2 montre le principe du test miscible visqueux avec un échantillon hétérogène "composite".

La figure 3 montre l'exemple d'un signal de pression différentielle DP attendu pour l'échantillon de la figure 2.

La figure 4 illustre l'injection du fluide 2 (F2) dans l'échantillon initialement saturé par le fluide 1 (F1).

La figure 5 montre une courbe de la pression différentielle (DP) mesurée en fonction du temps (T).

La figure 6 illustre un profil de perméabilité (K) calculé en fonction du temps T et un profil de perméabilité théorique.

La figure 7 montre un profil de perméabilité calculé en fonction de la distance et un profil de perméabilité théorique.

La figure 8 décrit les étapes utilisées pour déterminer une carte de perméabilité 3D en calant les pressions différentielles.

La figure 9 décrit les étapes utilisées pour déterminer une carte de perméabilité 3D en calant les pressions différentielles et les données de concentrations.

**Description détaillée de la méthode**

La méthode selon l'invention repose sur une approche intégrée combinant l'acquisition de données expérimentales rapides et non destructives pour l'échantillon et leur interprétation à l'aide d'un outil numérique dédié pour accéder à la carte 3D de perméabilité absolue. Selon la méthode, deux types de mesures expérimentales sont nécessaires :

- une mesure statique de la carte 3D de porosité de l'échantillon:

  Cette carte peut être calculée par exemple à partir de mesures RX (mesures par rayons X) obtenues avec des fluides de densités différentes saturant le milieu poreux. D'autres techniques comme la tomographie RMN permettent également d'obtenir cette carte de porosité. La valeur locale de la porosité peut être alors calculée de manière déterministe en utilisant la différence d'atténuation entre les signaux (RMN et/ou X) obtenus sous les deux conditions de saturation, à savoir pour l'échantillon sec et l'échantillon saturé. Une telle méthode est décrite par exemple dans le document suivant : Maloney, D. R., Wegener, D. C. "Significance absorption coefficients when determining in situ saturations by linear X-ray scans," International Symposium of the Society of Core Analysts, Socorro, USA, 2000.

- une mesure reposant sur au moins un déplacement miscible:

  Ce déplacement est réalisé en utilisant un fluide d'injection plus visqueux que le fluide en place (rapport de viscosité par exemple supérieur à 50 cP). Il peut être avantageux de réaliser les tests en injectant le fluide visqueux dans les deux sens pour affiner la précision au niveau de l'interprétation des résultats en terme de perméabilité. Cette mesure peut être la différence des pressions mesurée de part et d'autre de l'échantillon.

La méthode comporte ensuite une étape d'interprétation des résultats. Celle-ci est réalisée en deux étapes distinctes. La première étape permet d'obtenir un profil 1D de perméabilité absolue le long de l'échantillon en utilisant l'évolution de la pression différentielle mesurée au cours des tests de déplacement miscible visqueux. Ce profil 1D de perméabilité est utilisé dans la seconde étape d'interprétation en combinaison avec la carte 3D de porosité pour obtenir la carte 3D de perméabilité.

**Mise en oeuvre expérimentale**

1 - Détermination d'une carte tridimensionnelle de la porosité de l'échantillon

De nombreuses techniques permettant l'acquisition d'une carte 3D de porosité sont bien connues des spécialistes. On pourra se référer par exemple au document suivant:

Maloney, D. R., Wegener, D. C. "Significance absorption coefficients when determining in situ saturations by linear X-ray scans," International Symposium of the Society of Core Analysts, Socorro, USA, 2000.

2- Déplacements miscibles visqueux

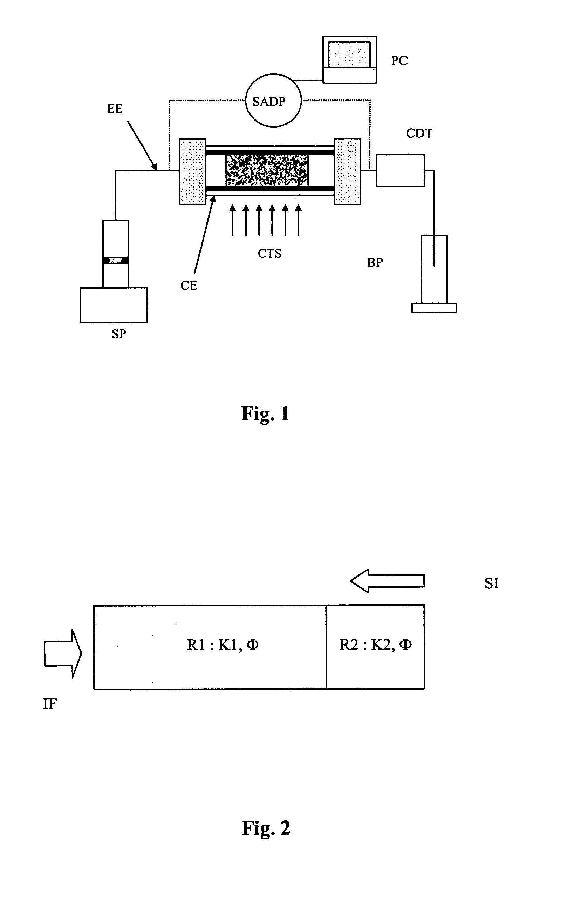

Le dispositif nécessaire est schématisé sur la figure 1. Il est constitué principalement d'une cellule porte échantillon (CE) classique munie ou non de prises de pression locale, d'un système de pompage (SP), et d'un système d'acquisition automatique (SADP) de la pression différentielle (DP) relié à ordinateur (PC). Un des points critiques est l'utilisation d'un capteur différentiel adapté à la gamme de perméabilité de l'échantillon testé. On peut positionner en sortie un conductimètre (CDT) pour suivre la production du fluide injecté en y ajoutant un traceur. Une burette de production (BP) peut être ajoutée en sortie du dispositif. L'évolution du fluide injecté au sein même de l'échantillon peut être obtenue par l'acquisition de mesures RX à l'aide d'un scanner (CTS) de type CT-scanner. Ce dernier dispositif peut en outre permettre de faire l'acquisition de la carte 3D de porosité dans le même environnement. Néanmoins, l'acquisition de ces différents types de mesure (conductimètre en sortie et profils intermédiaires de concentration en fluide injecté) n'est pas obligatoire pour analyser les données finales.

L'échantillon monté dans la cellule porte échantillon (CE) est saturé au départ avec un fluide de viscosité modérée (typiquement une saumure) que l'on appelle le fluide 1 dans la suite du document. On mesure alors sa perméabilité absolue moyenne à l'aide de la pompe (SP) et du capteur différentiel (SADP) en utilisant la loi de Darcy. Cette valeur de perméabilité est importante, car c'est elle qui dimensionne le test miscible visqueux en fonction du matériel disponible. Si on dispose par exemple d'un capteur différentiel (SADP) de 10 bars pleine échelle, on calcule à partir de cette valeur de perméabilité, le débit d'injection du fluide de viscosité élevée (appelé fluide 2 dans la suite) de manière à ce que la pression différentielle induite reste en deçà de la limite du capteur.

Avant le démarrage de l'injection du fluide 2, on laisse volontairement un volume mort dans l'embout d'entrée (EE) saturé avec le fluide 1 en place dans l'échantillon. Ce volume est très utile pour mesurer de façon précise le niveau de référence de la pression différentielle du fluide en place dans les conditions de l'expérience (débit). L'injection du fluide 2 se fait à débit imposé de préférence en utilisant une pompe sans pulsation pour éviter les artefacts au niveau du signal de pression. On utilise typiquement des débits d'injection de quelques centimètres cubes par heure. L'évolution de la pression différentielle au cours du test est mesurée de façon continue y compris pendant la période initiale de vidange du volume mort qui fournit précisément le niveau de référence de la pression différentielle pour le fluide 1. On injecte en général environ deux volumes poreux de fluide 2 pour être sûr d'obtenir un régime stabilisé en terme de pression différentielle. L'évolution de cette pression est mesurée de manière continue au cours des tests.

Une fois le test miscible visqueux terminé, le fluide 1 est remis en place dans l'échantillon par injection avec la pompe. L'évolution de la pression ou (et) le conductimètre permet(tent) alors d'évaluer le bon état de remise en place du fluide 1. Le déplacement étant ici fortement instable, on est beaucoup plus sensible aux hétérogénéités et il est nécessaire en général d'injecter un volume plus important pour retrouver l'état de base du fluide 1.

On réalise alors un second test qui suit exactement les mêmes procédures que le précédent, mais en procédant à l'injection dans le sens inverse.

## Interprétation des résultats

### Profil 1D de perméabilité

L'identification du profil 1D de perméabilité repose sur l'interprétation des tests précédemment décrits. La viscosité plus élevée du fluide 2 par rapport au fluide 1 déplacé assure en théorie un rapport de mobilité favorable, ce qui se traduit par un déplacement de type piston dans l'échantillon. Le temps de déplacement du front depuis l'entrée vers la sortie multiplié par le débit d'injection correspond alors au volume poreux de l'échantillon étudié. Ce calcul permet par ailleurs de convertir les temps d'expérience en positions du front de fluide 2 dans l'échantillon au cours de l'injection.

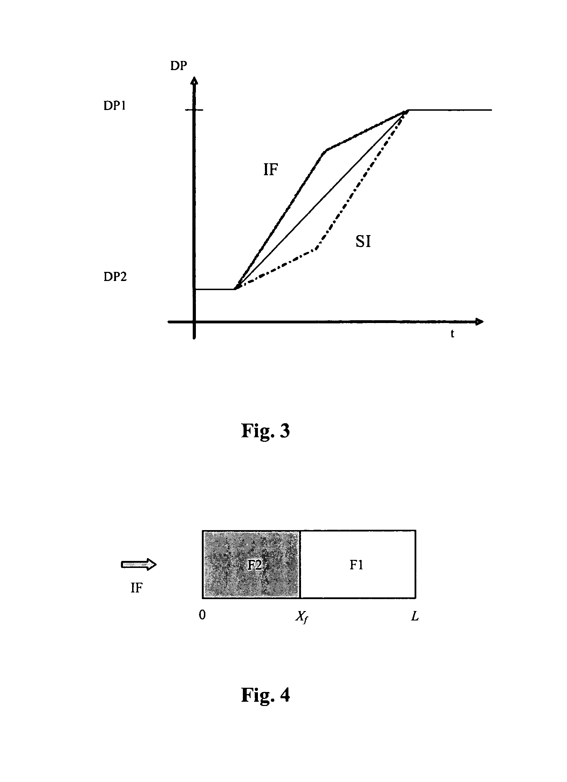

La loi de Darcy implique que pour un échantillon parfaitement homogène, l'évolution de la pression se fait de manière strictement linéaire avec une pente unique qui correspond à la valeur de la perméabilité. Pour un échantillon composite avec deux régions de perméabilité différentes, comme illustré sur la figure 2, on observe deux parties linéaires distinctes (figure 3). L'échantillon composite de la figure 2 est constitué d'une région R1 de perméabilité K1 inférieure à la perméabilité K2 de la seconde région R2.

La figure 3 illustre un exemple de signal de pression différentielle DP attendu pour l'échantillon composite de la figure 2. On note que la perméabilité est plus faible par rapport à la moyenne dans les 2 premiers tiers de l'échantillon. Dans le sens de l'injection du fluide miscible visqueux (IF), la courbe de pression différentielle DP est représentée en trait épais continu. La première pente est plus prononcée car c'est la région la moins perméable (R1) que le front visqueux traverse en premier. Ensuite, on observe un infléchissement de la pente dès lors que le front visqueux arrive au niveau de la région 2 plus perméable. Dans cet exemple théorique, la cassure entre les deux pentes est atteinte pour un temps qui n'est pas symétrique par rapport aux deux plateaux car la région 1 est plus longue et les porosités (Φ) sont égales. Le test inverse (SI) est représenté par une courbe discontinue. Ce test conduit à une signature complètement symétrique en terme de pression différentielle. La première pente suivie est la plus faible tandis que la cassure survient plus tôt, les plateaux restant les mêmes. DP1 représente la pression différentielle lorsque l'échantillon est entièrement saturé par le fluide injecté (fluide 2), et DP2 représente la pression différentielle lorsque l'échantillon est entièrement saturé par le fluide en place (fluide 1).

La pression différentielle DP(t) est la différence de pression le long de l'échantillon, et, compte tenu de la nature du test, elle dépend du temps t. Cette pression différentielle est la somme de la pression différentielle dans la partie de l'échantillon saturée par le fluide 2 et de la pression différentielle dans la partie de l'échantillon saturée par le fluide 1 :

$$DP(t) = \int_{0}^{X_f} \frac{Q\mu_2}{AK(x)}dx + \int_{X_f}^{L} \frac{Q\mu_1}{AK(x)}dx \qquad\qquad (1$$

$X_f$ est la position du front et dépend du temps. *L* est la longueur de l'échantillon. *Q* est le débit de fluide à travers la surface *A*, c'est-à-dire la surface de l'échantillon perpendiculaire à la direction d'écoulement. K est la perméabilité. Les viscosités respectives des fluides 1 et 2 sont notées $\mu_1$ et $\mu_2$. La figure 4 illustre l'injection du fluide 2 (F2) dans l'échantillon initialement saturé par le fluide 1 (F1). Le fluide 2 est injecté (IF), il sature l'échantillon entre les coordonnées longitudinales de 0 à $X_f$ Le fluide 1 sature l'échantillon entre les coordonnées longitudinales de $X_f$ à L (longueur de l'échantillon).

Si on se réfère à la méthode de Buckley-Leverett (1942) développée pour les déplacements immiscibles, la position du front est donnée par :

$$X_f = \frac{Q}{\phi A} t \qquad\qquad ( 2$$

où $\phi$ est la porosité. La dérivée de $X_f$ par rapport au temps est donc $Q/(\phi A)$.

On en déduit que la dérivée de $DP$ par rapport au temps est :

$$\frac{\partial DP}{\partial t} = \frac{Q^2}{A^2 \phi K(X_f)} (\mu_2 - \mu_1) \qquad\qquad ( 3$$

L'équation 2 donne la relation entre la position du front et le temps. On peut alors estimer la perméabilité en fonction du temps :

$$K(t) = \frac{Q^2}{A^2 \phi} (\mu_2 - \mu_1) \frac{1}{\dfrac{\partial DP}{\partial t}} \qquad\qquad ( 4$$

ou de façon équivalente la perméabilité le long de l'échantillon. Cette première étape permet de déterminer le profil 1D de perméabilité. Une valeur de perméabilité est représentative de la moyenne des perméabilités sur une tranche de l'échantillon, perpendiculaire à la direction d'écoulement.

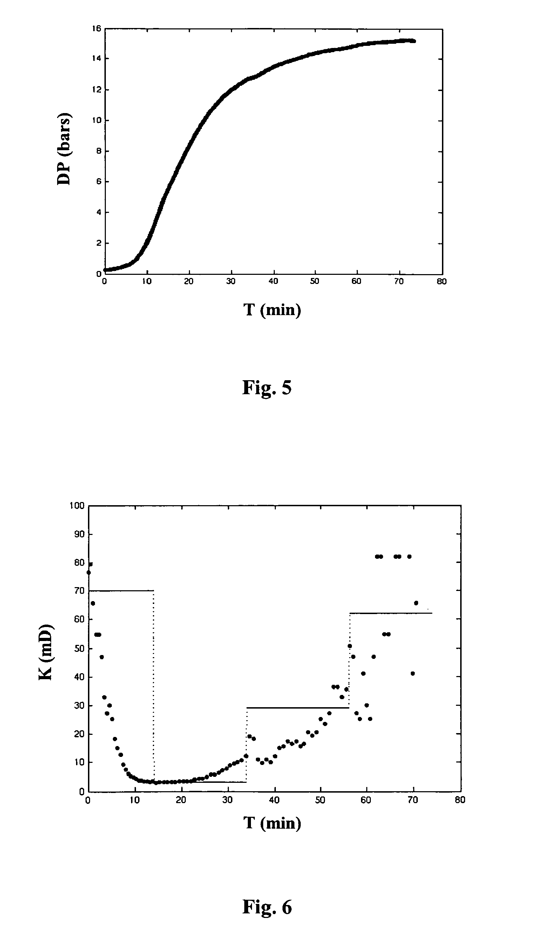

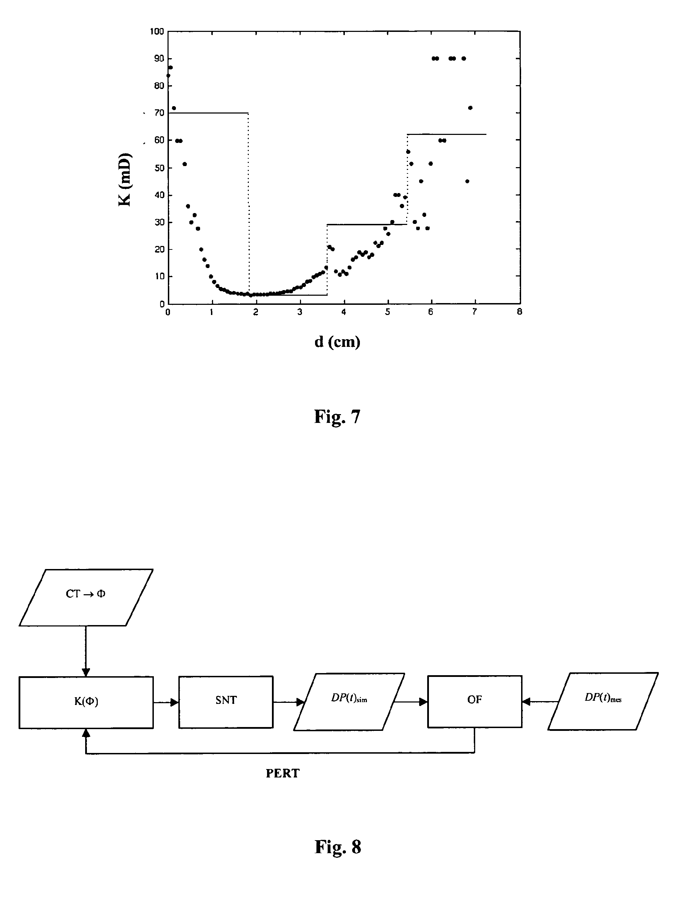

Afin d'illustrer cette première étape du processus de caractérisation 1D de la perméabilité, un échantillon a été construit en mettant bout à bout quatre échantillons différents, quasi-homogènes et de perméabilités effectives connues. L'échantillon a été initialement saturé par une saumure (30 g/l de NaCl). Puis, on y injecte de la glycérine à raison de 15 cm$^3$/h. La viscosité de la saumure est de 1,01 cP et celle de la glycérine de 60 cP. L'évolution de la pression différentielle (DP) au cours du temps (T) est représentée sur la figure 5. L'équation 4 permet d'en déduire le profil de perméabilité (K) en fonction du temps (T), illustrée sur la figure 6. Ce profil est comparé aux perméabilités effectives mesurées pour les 4 bouts d'échantillons. Les morceaux d'échantillons avaient été sélectionnés pour leur caractère homogène. Le profil calculé reproduit raisonnablement bien le profil théorique. La figure 7 montre le même profil en fonction de la longueur (d) de l'échantillon. Sur la figure 6, les points représentent le profil de perméabilité calculé en fonction du temps, et la courbe noire représente le profil de perméabilité théorique. De même, sur la figure 7, les points représentent le profil de perméabilité calculé en fonction de la distance, et la courbe noire représente le profil de perméabilité théorique.

<u>Carte 3D de perméabilité</u>

Le principe de l'interprétation repose sur une combinaison de résultats dynamiques (profil 1D de perméabilité) avec des résultats statiques (carte 3D de porosité). En effet, en supposant qu'il existe une loi porosité/perméabilité ($K(\phi)$) valable à l'échelle de la discrétisation retenue pour établir la carte 3D de porosité, il est alors possible de calculer une carte 3D de perméabilité a priori. En moyennant alors la perméabilité au niveau des différentes tranches (calcul en parallèle), on obtient alors une information directement comparable avec le profil 1D de perméabilité mesuré directement par interprétation de tests dynamiques. L'enjeu est alors d'optimiser la loi $K(\phi)$ de façon à obtenir la meilleure concordance possible entre les deux profils.

La figure 8 illustre les différentes étapes suivies pour obtenir une carte 3D de perméabilité cohérente avec les données disponibles, c'est à dire la carte 3D de porosité et la pression différentielle mesurée au cours du temps.

1) Dans un premier temps, on construit une carte 3D de perméabilité ($K(\Phi)$) à partir de la carte 3D de porosité (carte 3D de porosité $\Phi$ déduite de données CT). On peut se placer dans un cadre stochastique et procéder par co-simulation, auquel cas la carte de perméabilité dépend d'un germe et est corrélée à la porosité. On peut aussi employer une relation déterministe entre la porosité et la perméabilité. Par exemple, la loi $K(\phi)$ peut prendre la forme suivante : $K = \alpha (\phi - \phi_0)^\beta$

où :

α est un préfacteur,

$\phi_0$ la porosité résiduelle, *i.e.* une valeur de porosité pour laquelle l'écoulement ne peut plus se produire,

β un exposant traduisant l'évolution de la perméabilité en fonction de la porosité utile.

Dans le cadre de l'étude d'un milieu poreux présentant une structure plus complexe (bimodale par exemple), il est possible d'introduire deux lois $K1(\phi)$, $K2(\phi)$ mais aussi une valeur seuil de porosité $\phi_c$ permettant de distinguer les mailles où appliquer chacune des lois.

2) La carte 3D de perméabilité étant connue, on simule numériquement le test miscible visqueux (SNT). Les résultats de la simulation, c'est-à-dire la pression différentielle simulée $(DP(t)_{sim})$, sont alors comparés à la pression différentielle mesurée au cours du temps $(DP(t)_{mes})$. On en déduit la valeur d'une fonction objectif (OF). La fonction objectif OF mesure l'écart entre les données réelles et les réponses correspondantes simulées. On peut aussi envisager d'exprimer cette fonction différemment. Par exemple, elle pourrait mesurer l'écart entre le profil 1D de perméabilité déduit des données de pression du profil 1 D de perméabilité déduit des pressions simulées.

3) La dernière étape consiste à perturber (PERT) les paramètres du problème afin de déterminer une carte 3D de perméabilité qui minimise la fonction objectif, c'est-à-dire qui minimise l'écart entre la pression différentielle mesurée et le signal numérique correspondant.

Dans le cas d'une co-simulation, on modifiera la carte de perméabilité en appliquant par exemple la méthode de déformation graduelle. Dans le cas d'une relation déterministe, on modifiera les paramètres intervenant dans la loi, par exemple α, $\phi_0$ et β.

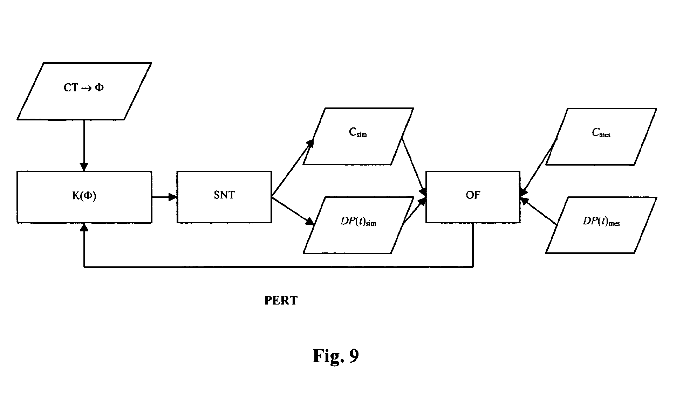

Ce processus de calage peut être enrichi par des mesures de concentrations (C). En effet, lorsque l'expérience se fait sous scanner, il est possible d'obtenir un profile de concentration ou encore des cartes 3D de concentration à des temps différents $(C_{mes})$ à partir de mesures RX (Maloney et Wegener, 2000). Ces données pourront être intégrées à la fonction objectif pour être comparées aux réponses simulées équivalentes $C_{sim}$ (figure 9).

Les applications potentielles de cette méthode sont importantes en particulier en ce qui concerne la caractérisation des milieux poreux hétérogènes comme les milieux vacuolaires par exemple, ou encore les milieux poreux soumis à des écoulements réactifs (suite à l'injection de CO2 ou à une opération de stimulation acide de puits par exemple).

## Revendications

1. Méthode de détermination d'une distribution tridimensionnelle de la perméabilité absolue d'un échantillon hétérogène, dans laquelle on effectue une mesure statique d'une carte de porosité tridimensionnelle dudit échantillon, **caractérisée en ce qu'**elle comporte les étapes suivantes :

   - on réalise au moins une expérience de déplacement miscible visqueux au cours de laquelle on détermine une évolution de la pression différentielle $DP(t)_{mes}$ de part et d'autre dudit échantillon ;
   - on détermine un profil 1D de perméabilité absolue le long dudit échantillon en utilisant ladite évolution de la pression différentielle ;
   - on construit une première carte 3D de perméabilité $(K(\Phi))$ à partir de ladite carte 3D de porosité ;
   - on estime une pression différentielle simulée $DP(t)_{sim}$, en simulant numériquement un test miscible visqueux à partir de ladite première carte de perméabilité et dudit profil 1D de perméabilité ;
   - on détermine ladite distribution tridimensionnelle de la perméabilité absolue dudit échantillon en modifiant au moins une fois ladite première carte de perméabilité de façon à minimiser l'écart entre ladite pression différentielle simulée $DP(t)_{sim}$ et ladite pression différentielle mesurée au cours du temps $DP(t)_{mes}$.

2. Méthode de détermination de la distribution 3D de la perméabilité absolue d'un échantillon hétérogène selon la revendication 1, dans laquelle on détermine ladite carte de porosité à partir de mesures statiques non destructives.

3. Méthode de détermination de la distribution 3D de la perméabilité absolue d'un échantillon hétérogène selon la revendication 2, dans laquelle on détermine ladite carte de porosité à partir de mesures par rayons X en utilisant des fluides de densités différentes saturant ledit échantillon.

4. Méthode de détermination de la distribution 3D de la perméabilité absolue d'un échantillon hétérogène selon la revendication 2, dans laquelle on détermine ladite carte de porosité à partir de mesures par résonance magnétique nucléaire.

5. Méthode de détermination de la distribution 3D de la perméabilité absolue d'un échantillon hétérogène selon la revendication 1, dans laquelle on détermine ladite première carte de perméabilité ($K(\Phi)$) à partir de co-simulations.

6. Méthode de détermination de la distribution 3D de la perméabilité absolue d'un échantillon hétérogène selon la revendication 1, dans laquelle on détermine ladite première carte de perméabilité ($K(\Phi)$) à partir d'au moins une relation déterministe entre la porosité et la perméabilité.

7. Méthode de détermination de la distribution 3D de la perméabilité absolue d'un échantillon hétérogène selon la revendication 5, dans laquelle on modifie ladite première carte de perméabilité en appliquant une méthode de déformation graduelle.

8. Méthode de détermination de la distribution 3D de la perméabilité absolue d'un échantillon hétérogène selon la revendication 1, dans laquelle on détermine en outre un profil de concentration ou des cartes 3D de concentration à des temps différents, que l'on compare à des réponses simulées équivalentes $C_{sim}$.

**Fig. 1**

**Fig. 2**

**Fig. 3**

**Fig. 4**

**Fig. 5**

**Fig. 6**

**Fig. 7**

**Fig. 8**

**PERT**

**Fig. 9**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | WO 03/071253 A (INST FRANCAIS DU PETROL [FR]; LENORMAND ROLAND [FR]; EGERMANN PATRICK) 28 août 2003 (2003-08-28) * page 3, ligne 19 - page 4, ligne 11 * * page 5, ligne 12-30; figure 1 * ----- | 1-8 | INV. G01N15/08 ADD. G01N33/24 |
| Y | US 5 146 086 A (DE BIBHAS R [US] ET AL) 8 septembre 1992 (1992-09-08) * colonne 1, ligne 22-38 * * colonne 6, ligne 7-49 * * colonne 13, ligne 54 - colonne 14, ligne 29; figure 3 * ----- | 1-8 | |
| A | US 4 868 751 A (DOGRU ALI H [US] ET AL) 19 septembre 1989 (1989-09-19) * colonne 2, ligne 25-44 * * colonne 5, ligne 31-68; figures 1,5 * ----- | 1-8 | |
| A | LIN Y. HU, MICKAELE LE RAVELEC-DUPIN: "An Improved Gradual Deformation Method for Reconciling Random and Gradient Searches in Stochastic Optimizations." MATHEMATICAL GEOLOGY, vol. 36, no. 6, août 2004 (2004-08), pages 703-719, XP002440346 * abrégé * ----- | 1,5-8 | DOMAINES TECHNIQUES RECHERCHES (IPC) G01N G01R |
| A | FR 2 863 052 A1 (INST FRANCAIS DU PETROL [FR]) 3 juin 2005 (2005-06-03) * page 2, ligne 26 - page 3, ligne 10 * * page 4, ligne 13-29; figure 5 * ----- | 1 | |
| A | EP 1 548 455 A (INST FRANCAIS DU PETROL [FR]) 29 juin 2005 (2005-06-29) * alinéas [0016] - [0018], [0022] - [0027]; figure 6 * ----- | 1 | |

Le présent rapport a été établi pour toutes les revendications

| Lieu de la recherche | Date d'achèvement de la recherche | Examinateur |
|---|---|---|
| La Haye | 12 mars 2008 | Wulveryck, J |

**ANNEXE AU RAPPORT DE RECHERCHE EUROPEENNE**
**RELATIF A LA DEMANDE DE BREVET EUROPEEN NO.**

EP 07 29 1405

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-03-2008

| Document brevet cité au rapport de recherche | | Date de publication | Membre(s) de la famille de brevet(s) | | Date de publication |
|---|---|---|---|---|---|
| WO 03071253 | A | 28-08-2003 | AU<br>BR<br>CA<br>EP<br>FR<br>MX<br>NO<br>US | 2003247372 A1<br>0303214 A<br>2474712 A1<br>1521957 A2<br>2836228 A1<br>PA04008018 A<br>20034691 A<br>2005178189 A1 | 09-09-2003<br>06-07-2004<br>28-08-2003<br>13-04-2005<br>22-08-2003<br>26-11-2004<br>19-12-2003<br>18-08-2005 |
| US 5146086 | A | 08-09-1992 | AUCUN | | |
| US 4868751 | A | 19-09-1989 | AUCUN | | |
| FR 2863052 | A1 | 03-06-2005 | GB<br>US | 2409730 A<br>2005229680 A1 | 06-07-2005<br>20-10-2005 |
| EP 1548455 | A | 29-06-2005 | CA<br>FR<br>US | 2491009 A1<br>2864238 A1<br>2005168220 A1 | 17-06-2005<br>24-06-2005<br>04-08-2005 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **DABBOUK, C ; ALI, L ; WILLIAMS, G ; BEATTIE, G.** Waterflood in vuggy layer in a Middle East reservoir - Displacement physics understood. *SPE 78530, Abu Dhabi International Petroleum Exhibition and Conference,* 13 Octobre 2002
- **OLIVIER, P ; CANTEGREL, L ; LAVEISSIÈRE, J ; GUILLONNEAU, N.** Multiphase flow behaviour in vugular carbonates using X-Ray. *SCA 2004-13, Society of Core Analysts Symposium,* 05 Octobre 2004
- **ZHAN, L ; YORTSOS, Y.C.** A direct method for the identification of the permeability field of an anisotropic porous medium. *SPE 62976, Annual Technical Conference and Exhibition,* 01 Octobre 2000
- **FINCHAM, A ; GOUTH, F.** Improvements of coreflood design and interpretation using a new software. *SCA 2000, Society of Core Analysts Symposium,* 09 Octobre 2000
- **MALONEY, D. R ; WEGENER, D. C.** Significance absorption coefficients when determining in situ saturations by linear X-ray scans. *International Symposium of the Society of Core Analysts,* 2000